# EUROPEAN PATENT APPLICATION

(11) **EP 2 163 192 A2**
(43) Date of publication of application: **17.03.2010**
(21) Application number: 09169956.1
(22) Date of filing: 10.09.2009
(51) Int. Cl.: A61B 5/0215

(54) **Intravascular Pressure Sensor**

(30) Priority: 16.09.2008 US 211592
(71) Applicant: Biosense Webster, Inc., Diamond Bar, CA 91765 (US)
(72) Inventor: Govari, Assaf, 34400, Haifa (IL); Ephrath, Yaron, 35170, Karkur (IL); Schwartz, Yitzhack, 34606, Haifa (IL)
(74) Representative: Brown, George Laurence

(57) **Abstract**

Pressure-sensing apparatus includes a sensor die, which is configured for percutaneous insertion through a wall of a blood vessel of a patient so as to generate an electrical signal that is responsive to a pressure in the blood vessel. A wire has a first end connected to the sensor die and a second end connected to an electronics package, which is configured for subcutaneous implantation and is connected via the wire to receive and process the electrical signal that is generated by the sensor die in order to provide an output that is indicative of the pressure.

## Description

The present invention relates generally to medical devices, and specifically to implantable pressure sensors.

To properly monitor and control conditions such as hypertension, it is desirable to measure intra-arterial pressure continuously. Existing intra-arterial pressure monitors, however, are for the most part not suitable for ambulatory use.

As one possible solution, U.S. Patent 6,053,873, describes a pressure-sensing stent. A flow parameter sensor is fixed to the stent and measures a parameter relating to a rate of blood flow through the stent. A transmitter transmits signals responsive to the measured parameter to a receiver outside the body.

As another example, U.S. Patent 6,939,299, describes an implantable miniaturized pressure sensor, which integrates a capacitor and an inductor in one small chip, forming a resonant LC circuit. The sensor is hermetically sealed and has a membrane that is deflected relative to the upper capacitor plate by an external fluid, gas, or mechanical pressure. The resonant frequency of the sensor can be remotely monitored and continuously measured with an external detector pick up coil disposed proximate the sensor. The pressure sensor may be used to measure intraocular pressure, intravascular pressure, intracranial pressure, pulmonary pressure, biliary-duct pressure, blood pressure, pressure in joints, and pressure in any body tissue or fluid.

Other types of implantable sensors that can be used for pressure measurements are described in U.S. Patent 6,802,811 and in U.S. Patent Application Publication 2002/0045921.

Embodiments of the present invention that are described hereinbelow provide a percutaneously-implantable intravascular pressure sensing device. The device comprises a pressure sensor die, which is inserted percutaneously through the wall of a blood vessel, and an electronics package, which is connected to the pressure sensor die by a wire and can be implanted immediately below the skin over the blood vessel. The electronics package may be powered and interrogated by a control unit outside the body, which is placed next to the skin above the package.

This sensing device enables continuous, accurate, ambulatory blood pressure monitoring, while minimizing leakage from the vessel and interference with normal blood flow. It can be introduced using a minimally-invasive procedure, which minimizes trauma to the blood vessel wall and surrounding tissues. The split design permits the pressure sensor to be made very small, while still providing a sophisticated electronics package, which can be accessed easily from outside the body. Unlike methods of ambulatory blood pressure monitoring that are known in the art, which are generally non-invasive, the minimally-invasive approach of embodiments of the present invention provide accurate, objective, continuous readings. This feature is of particular importance in monitoring patients with labile hypertension.

Although the embodiments that are described hereinbelow relate specifically to intra-arterial pressure measurement, the principles of the present invention may similarly be applied in measuring pressure inside other anatomical structures, such as veins and other body organs and passages.

There is therefore provided, in accordance with an embodiment of the present invention, pressure-sensing apparatus, including:
a sensor die, which is configured for percutaneous insertion through a wall of a blood vessel of a patient so as to generate an electrical signal that is responsive to a pressure in the blood vessel;
a wire having a first end connected to the sensor die and having a second end; an electronics package, which is configured for subcutaneous implantation and is connected to the second end of the wire so as to receive and process the electrical signal that is generated by the sensor die in order to provide an output that is indicative of the pressure.

Typically, the sensor die has a transverse outer dimension that is no greater than 1 mm.

In some embodiments, the apparatus includes an anchor, which is connected to the wire and is configured to open beneath skin of the patient following implantation of the sensing device in order to prevent accidental removal of the sensor die from the blood vessel.

Additionally or alternatively, the apparatus includes a control unit, which is configured to receive the output from the electronics package via a wireless link and to process the output so as to provide a reading of the pressure. In one embodiment, the device is configured to provide the reading of the pressure continuously and to automatically apply a therapy to the patient responsively to the pressure. The reading may be provided and the therapy applied by a control unit that is strapped to a limb of the patient.

In a disclosed embodiment, the apparatus includes a trocar for insertion through skin of the patient into proximity with the blood vessel; a puncture tool, which is configured to be inserted through the trocar and to make a hole through a wall of the blood vessel; and an inserter, which is configured to be inserted through the trocar after withdrawal of the puncture tool so as to insert the sensor die via the trocar through the hole into the blood vessel. The trocar may include a shaft having a longitudinal slot, wherein the wire passes through the slot while the sensor die is in the trocar so as to connect the sensor die to the electronics package outside the trocar.

There is also provided, in accordance with an embodiment of the present invention, a method for sensing pressure, including:
implanting a sensor die percutaneously through a wall of an anatomical structure in a body of a patient;
subcutaneously implanting an electronics package, which is connected to the sensor die by a wire;
processing in the electronics package an electrical signal received via the wire from the sensor die in order to provide an output that is indicative a pressure in the anatomical structure.

The sensor die may have a transverse outer dimension that is no greater than 1 mm. In one embodiment, the implanting the sensor die comprises opening an anchor, which is connected to the wire, beneath skin of the patient following implantation of the sensor die in order to prevent accidental removal of the sensor die from the blood vessel.

The method may further comprise receiving the output from the electronics package via a wireless link and processing the output so as to provide a reading of the pressure. The processing the output may comprise providing the reading of the pressure continuously, and wherein the method comprises automatically applying a therapy to the patient responsively to the pressure. The reading may be provided and the therapy applied by a control unit that is strapped to a limb of the patient

In some embodiments, implanting the sensor die includes inserting a trocar through skin of the patient into proximity with the anatomical structure; passing a puncture tool through the trocar so as to make a hole through a wall of the anatomical structure; and inserting the sensor die via the trocar through the hole into the anatomical structure.

In a disclosed embodiment, implanting the sensor die includes inserting the sensor die through the wall of an artery, such as a brachial artery. The artery may be visualized using ultrasound imaging prior to inserting the sensor die.

Embodiments of the invention will now be described by way of example with reference to the accompanying drawings, in which:
Fig. 1 is a schematic, pictorial illustration of a kit for implantation of an intravascular pressure sensing device, in accordance with an embodiment of the present invention;
Fig. 2 is a schematic, pictorial illustration showing a method for implantation of a pressure sensing device in the arm of a patient, in accordance with an embodiment of the present invention;
Figs. 3-5 are schematic, pictorial illustrations showing details of successive stages in a method for implantation of an intravascular pressure sensing device, in accordance with an embodiment of the present invention;
Fig. 6 is a schematic, pictorial illustration showing deployment of an intravascular pressure sensing device after implantation, in accordance with an embodiment of the present invention; and
Fig. 7 is a schematic pictorial illustration showing a control unit for intravascular pressure measurement, in accordance with an embodiment of the present invention.

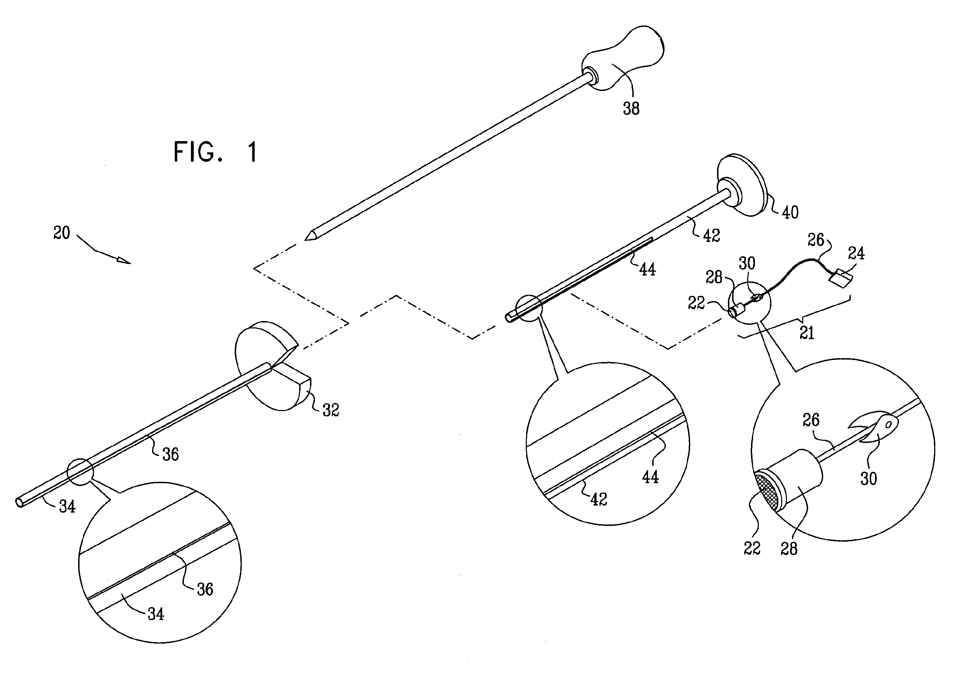

Fig. 1 is a schematic, pictorial illustration showing a kit 20 for use in implantation of an intravascular pressure sensing device 21, in accordance with an embodiment of the present invention.

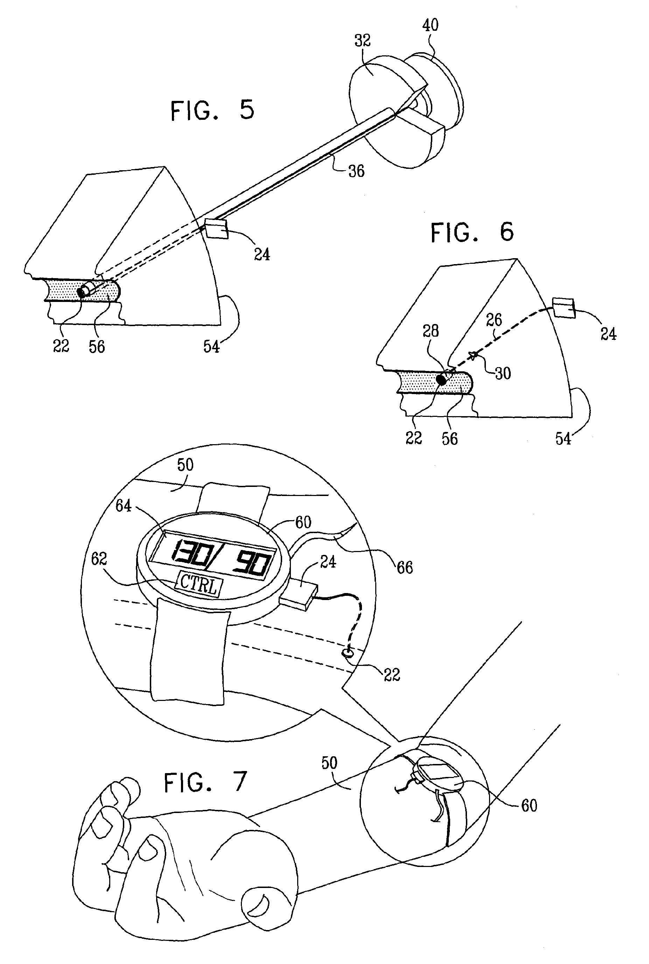

Device 21 itself comprises a sensor die 22, which is configured for percutaneous insertion through a wall of a blood vessel, as shown in the figures that follow. Sensor die 22 is connected by a wire 26 to an electronics package 24, which processes the electrical signal that is generated by the sensor die in order to provide an output that is indicative of the pressure in the blood vessel. To facilitate insertion through the vessel wall, the transverse outer dimension of the sensor die is typically less than 1 mm, although larger and smaller dies sizes may also be used depending on technological constraints and application requirements. The electronics package is made of a biocompatible material, suitable for implantation under the skin, and may be made in the form of a rectangle about 3-4 mm on a side and about 0.5-0.8 mm in height. The electronics package may be powered and interrogated by a control unit outside the body, which is placed next to the skin above the package, as shown in Fig. 7.

Sensor die 22 may comprise any suitable type of pressure-sensitive element. For example, the sensor die may comprise a capacitor, which deforms and thus varies its capacitance under pressure. The capacitor may be connected as part of a resonant circuit as described in the above-mentioned U.S. Patent 6,939,299, wherein the inductor and other elements of the resonant circuit may also reside in die 22 and/or in electronics package 24. The capacitance of the sensor die may then be measured by detecting the resonance peak in the circuit response. Alternatively, die 22 may comprise a piezoelectric element, which outputs a voltage signal to electronics package 24 in proportion to the pressure encountered by the die. Alternatively, die 22 may comprise any other suitable type of pressure-sensing element that is known in the art.

In the embodiment shown in Fig. 1, wire 26 includes an anchor 30 for tension relief after deployment of the sensing device in the patient's body. In addition, the wire may be connected to sensor die 22 by a safety release mechanism 28 to prevent the sensor die from being accidentally pulled out of the vessel wall after implantation.

Sensing device 21 is implanted percutaneously in a blood vessel using the components of kit 20 that are shown in Fig. 1, which include:
- A trocar 32, having a shaft 34 with a longitudinal slot 36 (for accommodating wire 26, as shown below).
- A puncture tool 38.
- An inserter 40, which also comprises a shaft 42 with a slot 44 for wire 26.

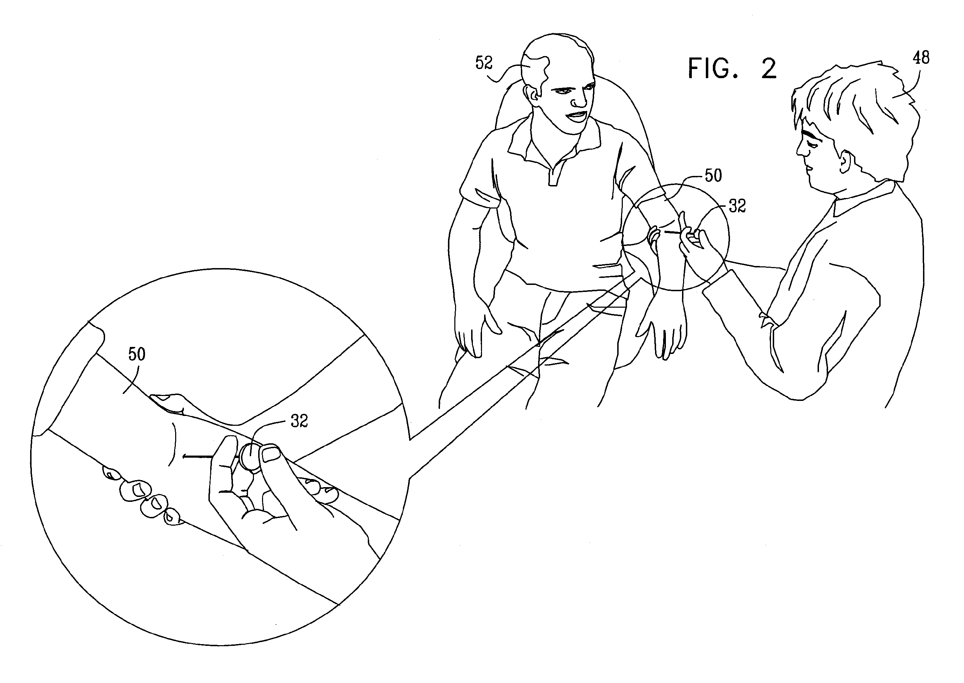

Fig. 2 is a schematic, pictorial illustration showing a medical practitioner 48 using elements of kit 20 to implant sensing device 21 in an arm 50 of a patient 52, in accordance with an embodiment of the present invention. In the pictured example, practitioner 48 is inserting the sensor die in the patient's brachial artery. This procedure may be performed under local anesthetic, possibly with the use of ultrasound imaging or other means to visualize the artery and avoid accidentally damaging nerves and other nearby structures, since in this percutaneous procedure, the practitioner is generally not able to see the target artery directly. The brachial artery is convenient because it is easily accessible by percutaneous approach. Alternatively, the devices and methods described herein may be applied to other arteries, as well as to veins and to measurement of pressure in other organs and body passages that are amenable to this sort of approach.

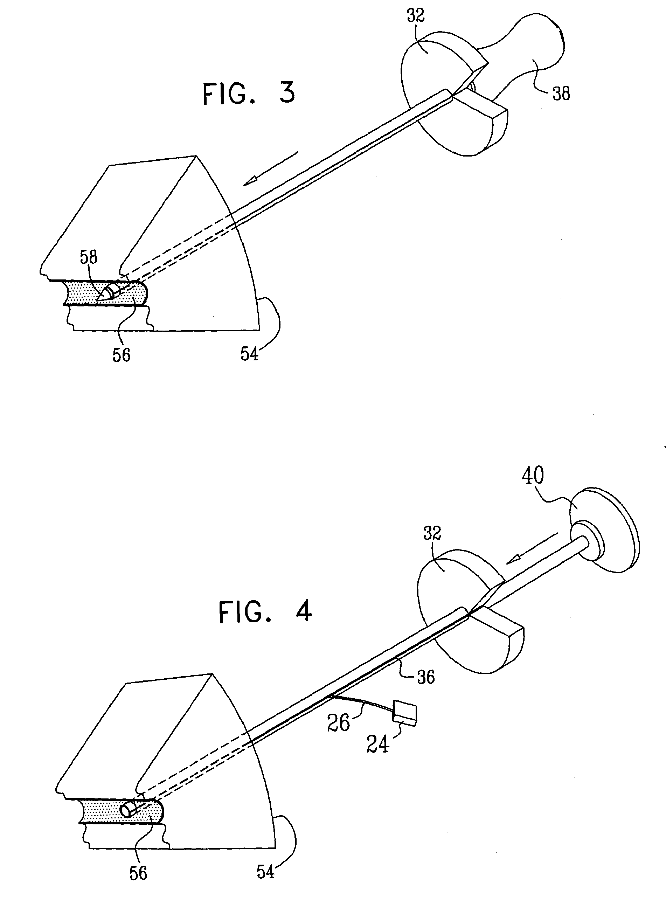

Figs. 3-5 are schematic, pictorial illustrations showing details of successive stages in implantation of sensing device 21 in a blood vessel 56, such as the brachial artery, in accordance with an embodiment of the present invention. First, as shown in Fig. 3, practitioner 48 passes the distal end of trocar 32 through skin 54 of patient 52 and through the underlying tissue until it reaches vessel 56. The practitioner then inserts puncture tool 38 through the shaft of the trocar and makes a small hole in the vessel wall using a distal point 58 at the end of the puncture tool.

Next, as shown in Fig. 4, the practitioner withdraws the puncture tool and uses inserter 40 to push the sensor die (which is not seen in this figure) through trocar 32 into vessel 56. The proximal end of wire 26 extends out to electronics package 24 through slots 44 and 36 (since the electronics package is too large to fit into the trocar shaft). At full extension, as shown in Fig. 5, sensor die 22 protrudes slightly into vessel 56, while electronics package 24 remains just above the surface of skin 54. The small size of the sensor die and the percutaneous approach to implantation that is illustrated here minimize trauma to the blood vessel and leakage of blood and promote rapid healing after implantation. The sensor die may be anchored in place within the blood vessel using an anchoring mechanism (not shown in the figures) that extends to the sides inside the vessel wall, such as a mechanism similar to that described in U.S. Patent 6,783,499, whose disclosure is incorporated herein by reference.

Fig. 6 is a schematic, pictorial illustration showing deployment of sensing device 21 after implantation, in accordance with an embodiment of the present invention. After sensor die 22 has been inserted through the wall of the blood vessel, as shown in Fig. 5, practitioner 48 withdraws inserter 40 and trocar 32 through skin 54. Withdrawal of the trocar causes anchor 30 on wire 26 near the sensor die to open outward, thus preventing the sensor from being accidentally pulled out of vessel 56. The practitioner may also implant electronics package 24 just under the surface of skin 54 through a suitable incision.

Fig. 7 is a schematic pictorial illustration showing a control unit 60 for intravascular pressure measurement in conjunction with sensor die 22 and electronics package 24, in accordance with an embodiment of the present invention. The control unit may, for example, be strapped around a limb of the body, such as arm 50, as shown in the figure, and may thus receive pressure readings from the sensor die (via the electronics package) continuously. Alternatively, the control unit may be placed next to the arm only intermittently, as needed. In either case, the control unit may transfer electrical power to electronics package 24 and receive output signals from the electronics package by wireless link, using induction, for example.

In the configuration shown in Fig. 7, a microcontroller 62 in control unit 60 interrogates electronics package 24 via the wireless link and processes the output of the electronics package to derive a calibrated pressure reading. This reading may be presented on a display 64. Alternatively or additionally, the reading may be stored in memory in the control unit and/or conveyed to a monitoring station (not shown) by a wireless or wired connection.

As another option, the continuous pressure monitoring provided by control unit 60 can be used to regulate closed-loop drug administration or other therapy for controlling hypertension. For example, in the embodiment shown in Fig. 7, control unit 60 comprises a drug reservoir and pump (not shown), which dispense medication through a tube 66 into the patient's body, with dosage based on the measured pressure.

It will be appreciated that the embodiments described above are cited by way of example, the scope of the present invention is defined by the appended claims and includes both combinations and subcombinations of the various features described hereinabove.

## Claims

1. Pressure-sensing apparatus, comprising:
a sensor die configured for percutaneous insertion through a wall of a blood vessel of a patient so as to generate an electrical signal that is responsive to pressure in the blood vessel;
a wire having a first end connected to the sensor die and having a second end;
an electronics package, which is configured for subcutaneous implantation and is connected to the second end of the wire so as to receive and process the electrical signal that is generated by the sensor die in order to provide an output that is indicative of the pressure.

2. The apparatus according to claim 1, wherein the sensor die has a transverse outer dimension that is no greater than 1 mm.

3. The apparatus according to claim 1 further comprising an anchor connected to the wire and configured to open beneath skin of the patient following implantation of the sensing device in order to prevent accidental removal of the sensor die from the blood vessel.

4. The apparatus according to claim 1 further comprising a control unit configured to receive the output from the electronics package via a wireless link and to process the output so as to provide a reading of the pressure.

5. The apparatus according to claim 4, wherein the device is configured to provide the reading of the pressure continuously and to automatically apply a therapy to the patient responsively to the pressure.

6. The apparatus according to claim 1 further comprising:
a trocar for insertion through skin of the patient into proximity with the blood vessel;
a puncture tool configured to be inserted through the trocar and to make a hole through a wall of the blood vessel; and
an inserter configured to be inserted through the trocar after withdrawal of the puncture tool so as to insert the sensor die via the trocar through the hole into the blood vessel.

7. The apparatus according to claim 6, wherein the trocar comprises a shaft having a longitudinal slot, and wherein the wire passes through the slot while the sensor die is in the trocar so as to connect the sensor die to the electronics package outside the trocar.

8. A method for sensing pressure, comprising:
implanting a sensor die percutaneously through a wall of an anatomical structure in a body of a patient;
subcutaneously implanting an electronics package, which is connected to the sensor die by a wire;
processing in the electronics package an electrical signal received via the wire from the sensor die in order to provide an output that is indicative a pressure in the anatomical structure.

9. The method according to claim 8, wherein the sensor die has a transverse outer dimension that is no greater than 1 mm.

10. The method according to claim 8, wherein implanting the sensor die comprises opening an anchor, which is connected to the wire, beneath skin of the patient following implantation of the sensor die in order to prevent accidental removal of the sensor die from the blood vessel.

11. The method according to claim 8, and comprising receiving the output from the electronics package via a wireless link and processing the output so as to provide a reading of the pressure.

12. The method according to claim 8, wherein implanting the sensor die comprises:
inserting a trocar through skin of the patient into proximity with the anatomical structure; passing a puncture tool through the trocar so as to make a hole through a wall of the anatomical structure; and
inserting the sensor die via the trocar through the hole into the anatomical structure.

13. The method according to claim 8, wherein implanting the sensor die comprises inserting the sensor die through the wall of an artery.

14. The method according to claim 13, wherein the artery comprises a brachial artery.

15. The method according to claim 13, wherein inserting the sensor die comprises visualizing the artery using ultrasound imaging prior to inserting the sensor die.
